# EUROPEAN PATENT APPLICATION

(11) **EP 2 011 529 A1**
(43) Date of publication of application: **07.01.2009**
(21) Application number: 08158769.3
(22) Date of filing: 23.06.2008
(51) Int. Cl.: A61M 1/14, A61J 1/10, A61M 1/00

(54) **Bag for haemodialysis that is reusable and to be hanged**

(30) Priority: 05.07.2007 IT MI20070237 U
(71) Applicant: HAEMOPHARM INDUSTRY AG, 9490 Vaduz (LI)
(72) Inventor: Valoti, Michele, 6900 Massagno (CH)
(74) Representative: Mittler, Enrico

(57) **Abstract**

Bag (1) for haemodialysis with at least a dialysing solution comprising on a first edge (4) hooking holes (6) for hanging the bag (1) and on a second edge (5) a mouth (3) for the exiting of the solution, characterised in that it has further hooking holes (7) placed on the second edge (5) in order to be able to hang the bag (1) upside down.

## Description

The present invention relates to a bag for haemodialysis that is reusable and to be hanged.

The dialysis operation involves withdrawing blood continuously from the patient and sending the blood to a special machine with a so-called dialysis filter, an element in which a semipermeable membrane of suitable porosity is used. Here, an exchange occurs between the blood and dialysing solution, in which the blood is purified of waste substances, which are transferred to the solution by osmosis, and enriched with the necessary nutritional elements, taken from the solution. The thus purified blood is then transfused again to the patient. The dialysing solution is generally contained in bags, typically made of plastics, which have a sealing edge. Along one side of this edge, holes are obtained for hanging the bag and enabling the outflow by the force of gravity through an opening obtained in the opposite edge towards the machine containing the dialysing filter. The dialysing solution consists of various types of ions in a water solution. Some of these types nevertheless have to be kept separate from one another and be mixed only at the moment of use, to avoid the precipitation of salts that are unusable for dialysis. For this purpose both bags with separating baffles that are breakable by hand are used for preparing the dialysing solution at the moment of use, and, in the case of compatibility between the ions, as explained above, also bags with a single component contained in a single compartment are used.

Normally, the emptied bag is thrown away. Nevertheless, in order to collect the used dialysing solution, comprising the reject substances of the blood, to be disposed of with suitable procedures, it is necessary to collect the used solution as it forms itself during haemodialysis in bags that are bought for that purpose.

In order to avoid waste in the provision of these disposal bags and for the disposal of the finished bags it was decided to make a useful modification that resolves this drawback and enables the initial bag to be reused.

The object of the present invention is to devise a bag for haemodialysis that is easily reusable for collecting the used dialysing solution.

According to the invention, this object is achieved by a bag for haemodialysis with at least a dialysing solution comprising on a first edge hooking holes for a hanging the bag and on a second edge a mouth for the exiting of the solution, characterised in that it has further hooking holes placed on the second edge in order to be hanged upside down.

These and other features of the present invention will be made clearer from the detailed description and an embodiment thereof illustrated by way of non-limiting example in the attached drawings, in which:
figure 1 shows a view of the in-use position of the bag for haemodialysis according to the invention;
figure 2 shows a schematic section view of figure 1 according to line II-II;
figure 3 shows a schematic section view of figure 1 according to line III-III;
figure 4 shows a bag for haemodialysis according to the invention in the reuse position.

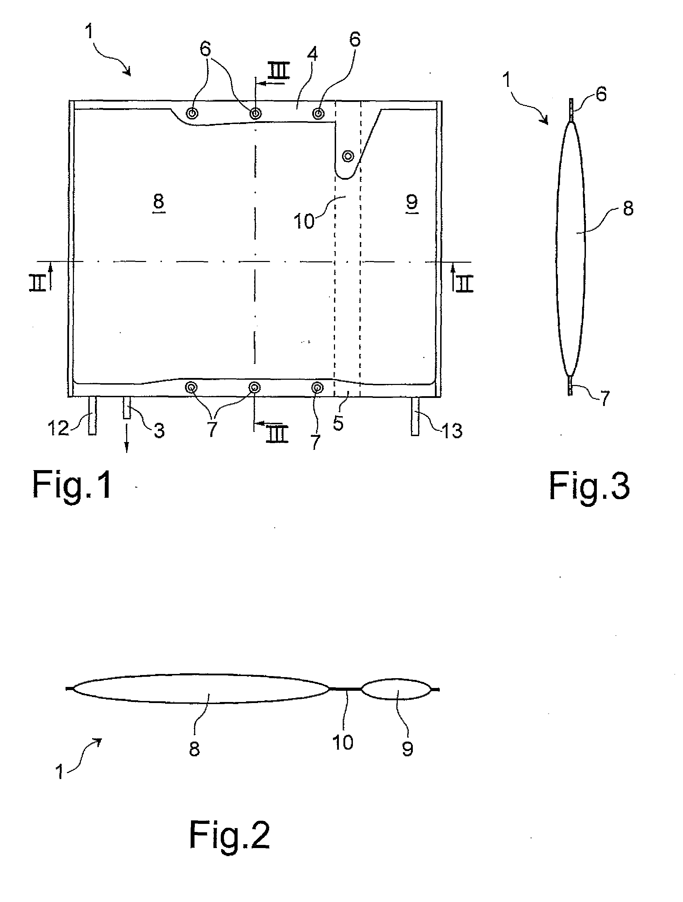

With reference to figures 1 to 4 there is known a bag 1 for haemodialysis containing a solution for haemodialysis formed of two ionic components that are kept separately until just before use, to prevent the formation of salts that are unusable for dialysis, a first component 8 and a second component 9, separated by a separating membrane 10 that is breakable by hand just before use. The bag 1 has two sealing edges: a first edge 4, located on the upper part of the bag 1 in first-use conditions, and a second edge 5, which in first-use conditions is placed on the lower edge. On the first edge 4 there are holes 6 for hanging the bag 1 in first-use conditions and enabling the solution 2 to descend by the force of gravity to the dialysis machine. The fresh solution 2 exits from a mouth 3 that is normally shut.

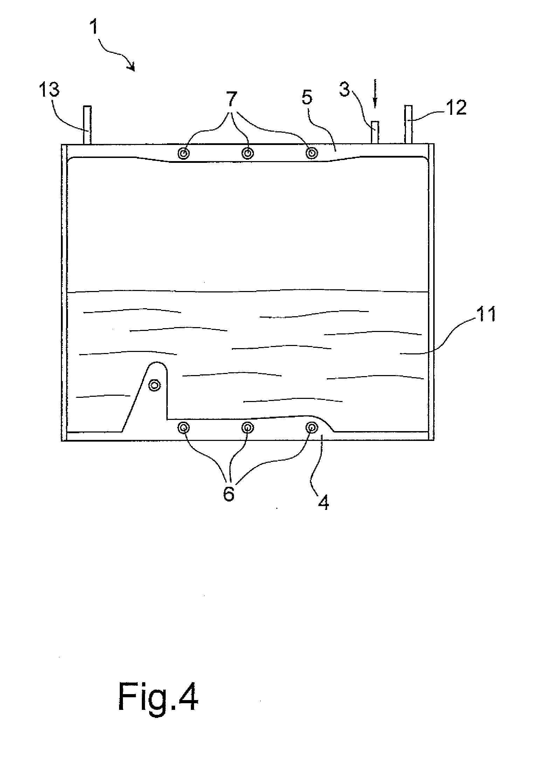

With reference now in particular to figure 4 on the second edge 5, further hooking holes 7 can be noticed that enable the used bag to be hanged upside down and enable it to be filled through the mouth 3 with a used dialysing solution 11 arriving by the force of gravity from the dialysis machine.

As already mentioned, in addition to the direct use method that provides for hanging the bag 1 by the holes 6 for supplying dialysing solution 2, a method is provided for reusing the bag 1 that, once it has been emptied for use, is hanged in an upside down position by the further hooking holes 7 and is reused to collect the used dialysing solution 11 through the force of gravity.

Advantageously, the bag 1 may have two further filling mouths 12 and 13 respectively for a first component 8 and a second component 9, sealed after filling is completed.

Advantageously, but not necessarily, the hooking holes 6 for hanging the bag 1 straight are three in number, and the further hooking holes 7 for appending the bag 1 for reuse are three in number.

## Claims

1. Bag (1) for haemodialysis with at least a dialysing solution comprising on a first edge (4) hooking holes (6) for hanging the bag (1) and on a second edge (5) a mouth (3) for the exiting of the solution, **characterised in that** it has further hooking holes (7) placed on the second edge (5) in order to be able to hang the bag (1) upside down.

2. Bag (1) according to claim 1, **characterised in that** it comprises a breakable inner separating membrane (10) for separating a first component (8) and a second component (9) of said solution until mixing thereof at the moment of use obtained by breaking said internal separating membrane (10).

3. Bag (1) according to claim 2, **characterised in that** it comprises two further filling mouths (12) and (13) respectively for the first component (8) and the second component (9), sealed after filling is completed.

4. Bag (1) according to any preceding claim, **characterised in that** the hooking holes (6) for hanging the bag (1) straight are three in number, and the further hooking holes (7) for appending the bag (1) for reuse are three in number.
